# EUROPEAN PATENT APPLICATION

(11) **EP 3 187 158 A1**
(43) Date of publication of application: **05.07.2017**
(21) Application number: 15836891.0
(22) Date of filing: 07.08.2015
(51) Int. Cl.: A61F 9/007

(54) **DEVICE PLACED UNDER EYELID**

(30) Priority: 28.08.2014 JP 2014174518
(71) Applicant: Universal View Co., Ltd., Tokyo 102-0093 (JP)
(72) Inventor: ARAI, Hiroyuki, Yokohama-shi Kanagawa 220-6204 (JP); MIKAWA, Sunao, Tokyo 102-0093 (JP); UEMURA, Eiichi, Tokyo 102-0093 (JP); SUZUKI, Yoshio, Tokyo 102-0093 (JP)
(74) Representative: D Young & Co LLP
(86) International application number: PCT/JP2015/072557
(87) International publication number: WO 2016/031535

(57) **Abstract**

The present invention has an object to provide an ocular insert device that can be stably placed under the eyelid without giving a feeling of insertion of a foreign body to a user and that can store a larger volume of drug as compared to a conventional system. The ocular insert device 100 includes a storage main body 1 that is formed to be a seed-shaped piece having a predetermined width so as to be located under an eyelid and that stores a drug. The storage main body 1 has a held portion 1a in which a surface that comes into contact with the eyelid can be held by a tarsal plate 52.

## Description

### [TECHNICAL FIELD]

The present invention relates to an ocular insert device which is preferably applicable to an ophthalmic drug delivery system (hereinafter, referred to as "DDS") that automatically exudes a drug to a site including an eyeball, a conjunctiva, etc.

### [BACKGROUND]

In recent years, a "punctal plug-type DDS", a "contact lens-type DDS", and the like have been developed in our country and others. The punctal plug-type DDS relates to a method in which DDS plugs containing a drug (hereinafter, referred to as "punctal plugs") are inserted into the upper and lower lacrimal puncta of each of the left and right eyes to exude the drug from the punctal plugs (see Patent Documents 1 to 3).

It is to be noted that some punctal plugs such as those disclosed in Patent Documents 1 to 3 contain an activator (drug). They can be used for treatment of glaucoma and the like by gently releasing the activator into the eye.

As described in Patent Document 4, the contact lens-type DDS also relates to a method in which a drug is gently released into the eye from a contact lens main body impregnated with the drug.

Patent Document 5 discloses an arc-shaped ophthalmic DDS device to be located in a lower cul-de-sac to exude a drug to the eyeball, the conjunctiva, etc.

### [DOCUMENTS FOR PRIOR ART]

### [PATENT DOCUMENTS]

Patent Document 1: Japanese Patent Application Publication No. 2008-049129
Patent Document 2: Japanese Patent Application Publication No. 2008-018234
Patent Document 3: Japanese Patent Application Publication No. 2008-006287
Patent Document 4: Japanese Patent Kohhyou Publication No. 2012-511395
Patent Document 5: US Patent No. 3,916,899

### [SUMMARY OF THE INVENTION]

### [PROBLEMS TO BE SOLVED BY THE INVENTION]

Meanwhile, the conventional ocular insert devices for use as the punctal plug-type DDS and the contact lens-type DDS have the following problems.
(i) In the case of the punctal plug-type DDS disclosed in Patent Documents 1 to 3, since lacrimal puncta are plugged which are originally the first exits for tears that flow through the nasolacrimal duct into the nose, normal tear drainage is prevented.
(ii) In the case of the punctal plug-type DDS, a head of the plug device sometimes comes into contact with the cornea on a nose side when the eyeball moves, which may cause a feeling of insertion of a foreign body.
(iii) In the case of the contact lens-type DDS disclosed in Patent Document 4, it is difficult to gently release a drug at a stable concentration because some users can produce sufficient tears but others cannot. Further, it is conceivable that a drug is deteriorated per se because the drug contained in the contact lens-type DDS main body is directly irradiated with light.
(iv) When the DDS device disclosed in Patent Document 5 is located in the upper cul-de-sac without any contrivances, a located posture of the device main body is unstable in an eyelid, and in addition, there is a fear that the device main body finally slips down toward a lower eyelid side.

Accordingly, this invention solves the above problems and has an object to provide an ocular insert device that can be stably placed under the eyelid without giving a feeling of insertion of a foreign body to a user and that can store a larger volume of drug as compared to a conventional system.

### [MEANS FOR SOLVING THE PROBLEMS]

In order to solve the above problems, the present invention claimed in claim 1 relates to an ocular insert device including a storage main body made of a semi-arc-shaped piece, the storage main body having a predetermined width so as to be located under an eyelid and having a shape which extends along a portion where a palpebral conjunctiva and a bulbar conjunctiva are connected together so as to supply a drug, wherein the storage main body includes a held portion surface in which a surface thereof coming into contact with the eyelid is held, and a surface thereof coming into contact with the eyeball with a smaller friction coefficient than that of the surface coming into contact with the eyelid.

According to the ocular insert device claimed in Claim 1, the surface of the held portion that comes into contact with the eyelid is brought into pressure contact with the tarsal plate after the device is placed, and therefore, the held portion can prevent the storage main body from slipping down. Since the surface of the storage main body that comes into contact with the eyeball has a smaller friction coefficient than that of the surface that comes into contact with the eyelid, it is possible for a user to reduce a feeling of insertion of a foreign body.

In the ocular insert device according to Claim 1, the invention claimed in Claim 2 relates to the ocular insert device wherein the held portion is extended along an outer edge of the storage main body so as to form a bulging shape, and is held by a tarsal plate.

In the ocular insert device according to Claim 1, the invention claimed in Claim 3 relates to the ocular insert device wherein the surface of the held portion that comes into contact with the eyelid includes a plurality of irregularities.

In the ocular insert device according to any one of Claims 1 to 3, the invention claimed in Claim 4 relates to the ocular insert device wherein the storage main body has a hollow portion, and the hollow portion has a bag shape with a predetermined capacity and has at least one opening formed in a predetermined position to exude a drug.

In the ocular insert device according to any one of Claims 1 to 3, the invention claimed in Claim 5 relates to the ocular insert device wherein the storage main body is formed as an impregnated structure in which the storage main body itself is impregnated with a drug.

### [EFFECTS OF THE INVENTION]

The ocular insert device according to the present invention allows the storage main body itself to be stably placed under the eyelid. It is also possible to store a larger volume of the drug as compared to a conventional system without giving a feeling of insertion of a foreign body to a user. Further, it is possible to reduce the number of parts. This enables a highly-reliable general-purpose ophthalmic drug delivery system (DDS) to be provided.

### [BRIEF DESCRIPTION OF THE DRAWINGS]

[FIG. 1A] FIG. 1A is a plan view of an ocular insert device 100 as a first embodiment of the present invention for showing a configuration example thereof.
[FIG. 1B] FIG. 1B is a sectional view of the ocular insert device 100, taken along the lines X1-X1 in FIG. 1A, for showing the configuration example thereof.
[FIG. 1C] FIG. 1C is a bottom view of the ocular insert device 100 for showing a configuration example thereof.
[FIG. 2] FIG. 2 is a sectional view showing an example where the ocular insert device 100 is located.
[FIG. 3A] FIG. 3A is a front view of a placement assistant tool 400 for showing a configuration example thereof.
[FIG. 3B] FIG. 3B is a plan view of the placement assistant tool 400 for showing the configuration example thereof.
[FIG. 3C] FIG. 3C is a side view of the placement assistant tool 400 for showing the configuration example thereof.
[FIG. 4A] FIG. 4A is a plan view of an ocular insert device 100' for showing a configuration example thereof.
[FIG. 4B] FIG. 4B is a front view of the ocular insert device 100' for showing the configuration example thereof.
[FIG. 5] FIG. 5 is a plan view showing an example where the ocular insert device 100' is located using the placement assistant tool 400.
[FIG. 6A] FIG. 6A is a front view of an ocular insert device 201 as a second embodiment of the present invention for showing a configuration example thereof.
[FIG. 6B] FIG. 6B is a side view of the ocular insert device 201 for showing the configuration example thereof.
[FIG. 6C] FIG. 6C is a plan view of the ocular insert device 201 for showing the configuration example thereof.
[FIG. 6D] FIG. 6D is a sectional view of the ocular insert device 201, taken along the line X2-X2 in FIG. 6C, for showing the configuration example thereof.
[FIG. 6E] FIG. 6E is a bottom view of the ocular insert device 201 for showing the configuration example thereof.
[FIG. 7A] FIG. 7A is a front view of an ocular insert device 202 for showing a configuration example thereof.
[FIG. 7B] FIG. 7B is a side view of the ocular insert device 202 for showing the configuration thereof.
[FIG. 7C] FIG. 7C is a plan view of the ocular insert device 202 for showing the configuration example thereof.
[FIG. 7D] FIG. 7D is a bottom view of the ocular insert device 202 for showing the configuration example thereof.
[FIG.8A] FIG. 8A is a plan view of an ocular insert device 203 for showing a configuration example thereof.
[FIG. 8B] FIG. 8B is a bottom view of the ocular insert device 203 for showing the configuration example thereof.
[FIG. 9] FIG. 9 is a sectional view showing an example where the ocular insert device 201 is located.
[FIG. 10A] FIG. 10A is a plan view of an ocular insert device 300 as a third embodiment of the present invention for showing a configuration example thereof.
[FIG. 10B] FIG. 10B is a plan view showing an example where the ocular insert device 300 is located.

### [EMBODIMENTS FOR CARRYING OUT THE INVENTION]

The following will describe an ocular insert device according to the present invention with reference to the drawings.

### <First Embodiment>

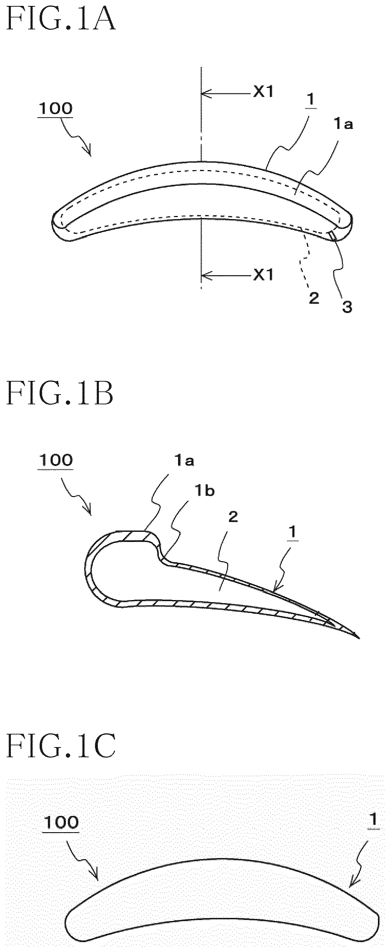

An ocular insert device 100 shown in FIGS. 1A to 1C includes a semi-arc and sheet-shaped storage main body 1 that can be located under the eyelid, and the storage main body 1 has a hollow portion 2. The ocular insert device 100 is intended to automatically supply eye drops to a site including the eyeball, the conjunctiva, etc. for a long time. The ocular insert device 100 is intended that the storage main body itself is deeply placed into the palpebral conjunctival sac, without depending on attachment to a C-shaped base body like the one in an ocular insert device which the present applicant has separately applied.

The conjunctiva of a human is separated into the palpebral conjunctiva and the bulbar conjunctiva, but they are respectively folded back deeply under the eyelid and are connected together. A shape extending along these portions is a seed shape, and in the present invention, it is characterized that the storage main body 1 is formed to have the seed shape. As an exterior shape of the storage main body 1, a shape such that the hollow portion 2 is not exposed at the inner corner of the eye is preferable. Therefore, in the present invention, the storage main body 1 is configured to have a seed-shaped structure that is one example of an arc-shaped piece having a predetermined width. The hollow portion 2 contains a predetermined amount of ophthalmic solutions.

The storage main body 1 has a held portion 1a in which a surface that comes into contact with the eyelid is held by the tarsal plate. For example, as shown in FIG. 1A, the held portion 1a is provided along the outer edge of the storage main body 1 so as to have a bulging shape. As shown in FIG. 1B, a cross section thereof indicates, for example, a wedge shape. It is formed that a stepped portion 1b of the wedge shape is brought into pressure-contact with the tarsal plate. The held portion 1a allows the storage main body itself to be prevented from slipping down after the ocular insert device 100 is located under the eyelid. A back surface of the storage main body 1 shown in FIG. 1C is subjected to mirror finishing (processing) for making the friction coefficient of the back surface smaller than that of the surface that comes into contact with the eyelid in order to reduce a feeling of insertion of a foreign body. The mirror finishing means that a metallic surface or a coating surface is finished like a mirror so as to reflect objects. This processing is performed to improve lubricity between the back surface of the storage main body 1 and the sclera.

In this embodiment, as shown in FIG. 1B, the hollow portion 2 is formed by providing a cavity in the storage main body 1, which makes it possible to encapsulate a drug in the storage main body 1. For example, the hollow portion 2 has a bag shape with a predetermined capacity and has at least one opening 3 provided in a predetermined position to automatically exude a drug. The opening 3 has a diameter of about several micrometers through several hundred micrometers to exude the drug for a long time.

The automatic exudation of a drug utilizes an osmotic phenomenon. The osmotic phenomenon refers to a phenomenon in which when two solutions of different concentrations are in contact with each other through a semipermeable membrane having a quality that is impermeable to a solute but permeable to a solvent, the solvent of the low-concentration solution diffuses into the high-concentration solution. A pressure produced by such an osmotic phenomenon is referred to as an osmotic pressure. For example, when two solutions of different concentrations are separated by a cell membrane, a force generated by transfer of water from the low-concentration solution to the high-concentration solution is referred to as the osmotic pressure.

### <Drugs>

As the drug(s) contained in the storage main body 1, an allergy drug can be used. For examples, azelastine HCl, emadastine difumerate, epinastine HCl, ketotifen fumerate, levocabastine HCl, olopatadine HCl, pheniramine maleate, and antazoline phosphate are exemplified. Further, a mast cell stabilizing agent can be used. For example, cromolyn sodium, lodoxamide tromethamine, nedocromil sodium, and permirolast potassium are exemplified.

As the drug(s), a mydriatic agent and a cycloplegic agent can be used. For example, henylephrine, atropine sulfate, homatropine, scopolamine HBr, cyclopentolate HCl, tropicamide, and phenylephrine HCl are exemplified. Further, an ophthalmic dye can be used. For example, rose bengal, sissamine green, indocyanine green, fluorexon, and fluorescein are exemplified.

As the drug(s), a corticosteroid (adrenal cortical steroid) can be used. For example, dexamethasone sodium phosphate, dexamethasone, fluoromethalone, fluoromethalone acetate, loteprednoletabonate, prednisolone acetate, prednisolone sodium phosphate, medrysone, rimexolone, and fluocinolone acetonide are exemplified. Further, a non-steroidal anti-inflammatory agent can be used. For example, flurbiprofen sodium, suprofen, diclofenac sodium, ketorolac tromethamine, ciclosporin, rapamycin methotrexate, azathioprine, and bromocriptine are exemplified.

As the drug(s), an anti-infective agent can also be used. For example, tobramycin, moxifloxacin, ofloxacin, gatifloxacin, ciprofloxacin, gentamicin, sulfisoxazole diolamine, sodium sulfacetamide, vancomycin, polymyxin B, amikacin, norfloxacin, levofloxacin, sulfisoxazole diolamine, sodium sulfacetamide tetracycline, doxycycline, dicloxacillin, cefalexin, amoxicillin/clavulanic acid, ceftriaxone, cefixime, erythromycin, ofloxacin, azithromycin, gentamycin, sulfadiazine, and pyrimethamine are exemplified.

As the drug(s), a drug for glaucoma can be used. As the drug for glaucoma, for example, nipradilol, levobunolol, dipivefrine, distigmine, bunazosin, isopropyl unoprostone, travoprost, tafluprost, epinephrines, α-2-adrenergic receptors (agonistic receptors), β blockers, direct miotics, cholinesterase inhibitors, carbonic anhydrase inhibitors, prostoglandins, and prostamides are exemplified. Epinephrines include dipivefrin and the like. Alpha-2-adrenergic receptors (agonistic receptors) include aproclonidine, brimonidine, and the like. β blockers include betaxolol, carteolol, levobunolol, metipranolol, timolol, and the like. Direct miotics include carbachol, pilocarpine, and the like. Cholinesterase inhibitors include physostigmine, echothiophate, and the like. Carbonic anhydrase inhibitors include acetazolamide, brinzolamide, dorzolamide, methazolamide, and the like. Prostoglandins and prostamides include latanoprost, bimatoprost, uravoprost, unoprostonecidofovir, and the like.

As the drug(s), a drug for cataract can be used. As the drug for cataract, for example, pirenoxine and glutathione are exemplified.

As the drug(s), an antiviral agent can also be used. For example, fomivirsen sodium, foscarnet sodium, ganciclovir sodium, valganciclovir HCl, trifluridine, aciclovir, and famciclovir are exemplified. Further, a local anesthetic agent can also be used. For example, tetracaine HCl, proparacaine HCl and fluorescein sodium, benoxinate and fluorescein sodium, benoxinate, and fluorexon disodium are exemplified. Further, an antifungal agent can also be used. For example, fluconazole, flucytosine, amphotericin B, itraconazole, and ketoconazole are exemplified.

As the drug(s), an analgesic agent can be used. For example, acetaminophen and codeine, acetaminophen and hydrocodone, acetaminophen, ketorolac, ibuprofen, and tramadol are exemplified. Further, a vasoconstrictor can also be used. For example, ephedrine hydrochloride, naphazoline hydrochloride, phenylephrine hydrochloride, tetrahydrozoline hydrochloride, and oxymetazoline are exemplified (but it is not limited thereto). Further, a vitamin, an antioxidant, and a nutraceutical product can also be used. For example, vitamin A, vitamin D, vitamin E, lutein, taurine, glutathione, zeaxanthin, and fatty acids are exemplified (but they are not limited thereto).

The drug contained in the storage main body 1 may contain an excipient. As the excipient, a synthetic or natural polymer may be used. As such a polymer, for example, polyvinyl alcohol, polyethylene glycol, polyacrylic acid, hydroxymethyl cellulose, glycerin, hypromelos, polyvinylpyrrolidone, carbopol, propylene glycol, hydroxypropyl guar, glucam-20, hydroxypropyl cellulose, sorbitol, dextrose (glucose), polysorbate, mannitol, dextran, modified polysaccharide and gum, phosolipid, and sulphobetain can be used.

As a material for the storage main body 1, for example, an ultraviolet absorber- and pig collagen-containing HEMA, a cross-linked acrylic ester copolymer, an ultraviolet absorber-containing acrylic-methacrylic cross-linked copolymer, an ultraviolet absorptive yellow soft acrylic resin, an ultraviolet absorptive high refractive index silicone, polyvinylidene fluoride (PVDF), an ethyl methacrylamide-based blue light absorber, a ultraviolet and blue light absorber-containing acrylic resin, and a polymethylmethacrylate resin can be used.

### <Production Method>

For example, in the case of production of the bag-shaped ocular insert device 100, although not shown, a mold including a cavity molded into a seed shape and a bulging shape (held portion) and a core is formed. Then, a silicone resin endless tube is set in the mold under aseptic conditions, and air is blown from the other end to form the silicone resin into a seed shape by balloon molding. Then, the air inlet is closed. When a drug is introduced into the hollow portion 2, the inside of the hollow portion 2 is once maintained under vacuum and then an opening for injecting a drug is formed, or an opening for injecting a drug is formed when the hollow portion 2 is in a non-vacuum state.

A predetermined amount of drug is injected through the opening, and then the opening is closed by a plug member. The drug may have any form such as liquid, powder, or jelly. Then, at least one opening 3 for exuding a drug from a predetermined position are formed. As the plug member, the same material as that of the storage main body 1 may be used. In this way, the drug-containing ocular insert device 100 can be formed.

For the storage main body 1, a porous material may be used. For example, instead of providing the hollow portion 2, the storage main body 1 may be formed as an impregnated structure by impregnating the storage main body 1 with a drug. When the material of the storage main body 1 itself is kneaded with a drug, it is possible to directly use the sheet-shaped storage main body 1 without providing a cavity in the storage main body 1. This allows a feeling of a foreign body to be more reduced when the ocular insert device 100 is inserted into the conjunctival sac.

The storage main body 1 constituted from an impregnated member may be produced by mixing a drug into the constituent material of the storage main body 1. Alternatively, a drug may be added to the storage main body 1 by immersing the previously-molded storage main body 1 in the drug.

The surface of the storage main body 1 is coated with a material impermeable to a drug so that the drug can be released from only any optionally uncoated portions of the storage main body 1. As the coating material, ethylene vinyl alcohol, ethylene vinyl acetate, cellulose derivatives, cellulose acetate, polydimethylene siloxane derivatives, and polyurethane can be used.

The storage main body 1 may be coated with a material (membrane) that is permeable to water but impermeable to a drug. When the storage main body 1 is inserted under the eyelid, water diffuses through the membrane into the storage main body 1 to create an osmotic gradient. This is described in US Patent Nos. 6,923,880 and 5,817,335. These US patents are incorporated herein by reference in their entireties. Then, the drug passes through an uncoated portion of the storage main body 1 by the osmotic gradient. The membrane may be made of a material containing, for example, HYTREL (registered trade mark) polybutylene terephthalate elastomer, cellulose ether, cellulose ester, water flux-enhancing polyvinyl acetate copolymer, or ethylene vinyl alcohol.

The storage main body 1 may be coated with a polymeric material having any thickness. In this case, the polymeric material contains at least one species of drug and the storage main body 1 is impermeable to the drug.

The storage main body 1 constituted from an impregnated member may be made of a polymeric material that is at least partially water-soluble. The appropriate polymeric material that is at least partially water-soluble includes phosolipids, polysulfobetains, polysaccharides, carbohydrates, proteins, and polyamino acids but is not limited thereto. Phosolipids include poly(ethylene glycol), poly(ethylene oxide), poly(propylene glycol), poly(vinyl alcohol), poly(hydroxyethyl methacrylate), poly(vinylpyrrolidone), polyacrylic acid, poly(ethyloxazoline), poly(dimethylacrylamide), and phosphoryl choline derivatives. Polysaccharides include hyaluronic acid, dextran, hydroxyethyl cellulose, hydroxypropyl cellulose, gellan gum, guar gum, heparan sulfate, chondroitin sulfate, heparin, and alginic acid. Proteins include gelatin, collagen, albumin, and ovalbumin. The polymeric material may be copolymerized or mixed with at least one of hydrophobic polymers and monomers.

The storage main body 1 constituted from an impregnated member may be made of a biodegradable polymeric material. The biodegradable polymeric material is preferably hydrolyzed under in vivo conditions. As the biodegradable polymeric material, polymers of glycolide, lactide, epsilon-caprolactone, and other hydroxylic acids may be used. Alternatively, oligomers or non-toxic or other biodegradabe polymers may also be used. Poly(alpha-hydroxylic acid) include poly(glycolic acid), poly(2-dioxanone), poly(DL-lactic acid), and poly(L-lactic acid). Other useful materials preferably include poly(amino acids), polycarbonates, poly(anhydrides), poly(orthoesters), poly(phosphazines), and poly(phosphates). Polylactones such as poly(epsilon-caprolactone), poly(delta-caprolactone), poly(delta-valerolactone), and poly(gamma-butyrolactone) are also useful. The same is true of chitosan, alginic acid, collagen, and gelatin. The polymeric material may be composed of a mixture of one or more dissoluble and biodegradable polymers.

The storage main body 1 constituted from an impregnated member may be made of a water-insoluble and non-biodegradable polymeric material. The polymeric material in this case includes cross-linked polymers. The cross-linked polymers include cross-linked poly(ethylene glycol), poly(ethylene oxide), poly(propylene glycol), poly(vinyl alcohol), poly(hydroxyethyl methacrylate), poly(vinylpyrrolidone), polyacrylic acid, poly(ethyloxazoline), and poly(dimethylacrylamide). These polymers may be copolymerized or mixed with at least one of hydrophobic polymers and monomers. As the water-insoluble and non-biodegradable polymer, silicone, silicone blends, silicone copolymers, silicone hydrogel polymers, phospholipids, polysulfobetains, polysaccharides, carbohydrates, proteins, polyamino acids, fluoropolymers, PTFE, PVDF, Teflon (registered trade mark), polypropylene, polyethylene, nylon (registered trade mark), and EVA (i.e., ethylene-vinyl acetate copolymer) and the like can be used. Silicone hydrogel polymers include hydrophilic monomers of pHEMA (polyhydroxyethyl methacrylate), polyethylene glycol, polyvinyl pyrrolidone, glycerol, silicone hydrogel polymers and the ones described in US Patent Nos. 5,962,548, 6,020,445, 6,099,852, 6,367,929, and 6,822,016 (these US Patents are incorporated herein by reference in their entireties). Phospholipids include phosphoryl choline derivatives. Proteins include hyaluronic acid, dextran, hydroxyethyl cellulose, hydroxypropyl cellulose, gellan gum, guar gum, heparan sulfate, chondroitin sulfate, heparin and alginic acid, gelatin, collagen, albumin, and ovalbumin. As the polymer that has at least one of water insolubility and non-biodegradability, silicone, polyurethane, cyanoacrylate, polyacrylic acid, fibrin, cross-linked proteins, albumin, and collagen-gelatin may also be used.

### <Handling Method>

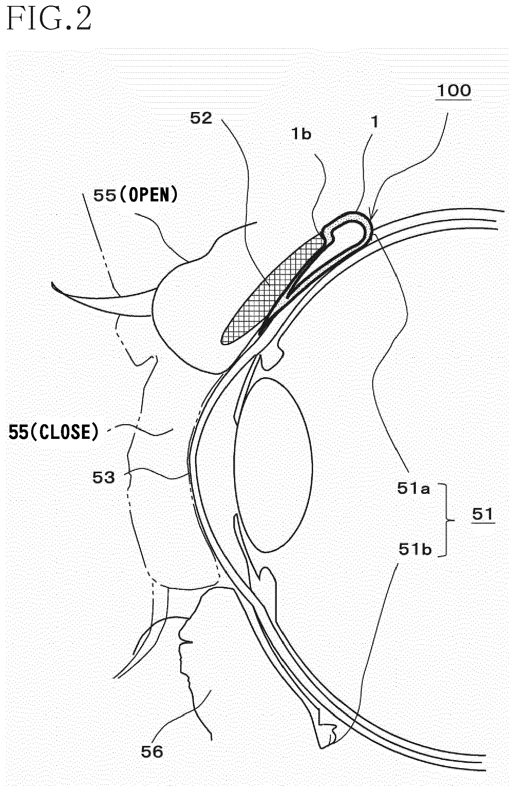

Here, a method for handling the ocular insert device 100 will be described with reference to FIGS. 2 to 5. The ocular insert device 100 according to the present invention may be inserted into either an upper cul-de-sac 51a on a side of an upper eyelid 55 or a lower cul-de-sac 51b on a side of a lower eyelid 56 in the eye of a user shown in FIG. 2. When a high-concentration drug needs to be used for treatment, the concentration of the drug can also be controlled by inserting the ocular insert devices 100 into both the cul-de-sacs.

In FIG. 2, a tarsal plate 52 exists on a position such that it covers an upper portion of a cornea 53 at a side of the upper eyelid 55 when the eye is closed. When the eye is opened, an upper end of the tarsal plate 52 acts to hold the stepped portion 1b of the storage main body 1 at a deep side of the upper cul-de-sac 51a. By utilizing such an action, the ocular insert device 100 can be placed in the upper cul-de-sac 51a, and the storage main body 1 of the ocular insert device 100 can be pressed down by the tarsal plate 52.

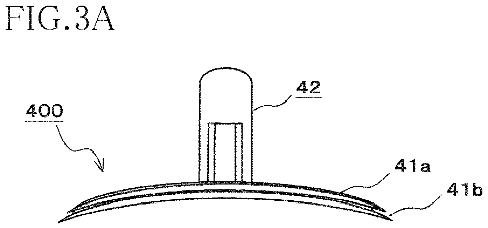

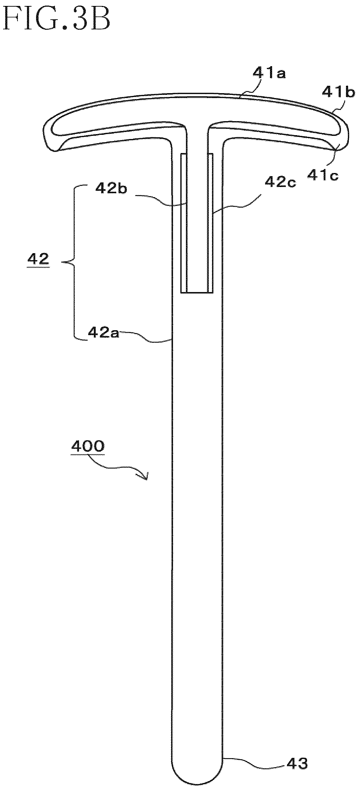

In order to easily locate the storage main body 1 into the eye, the ocular insert device 100 according to the present invention is provided with a T-shaped placement assistant tool 400, shown in FIG. 3A, having the function of tweezers. The placement assistant tool 400 has, for example, supporting parts 41a and 41b, the entire of which is made of a resin or stainless steel, and a holding part 42 having the structure of tweezers. As shown in FIG. 3B, the supporting parts 41a and 41b have a seed shape and are intended to pinch and support the storage main body 1. The supporting part 41b has barbs 41c each having an R-shaped cross section so that the barbs 41c receive an inner periphery of the storage main body 1.

The holding part 42 has a supporting point 43 and pinching plate-like gripping portions 42a and 42b, which constitute the system of tweezers, and is configured to hold the supporting parts 41a and 41b. The gripping portion 42a has a rectangular opening 42c, and the other gripping portion 42b passes through the opening 42c so that the gripping portions 42a and 42b laterally exchange their positions. A tip of the gripping portion 42a constitutes the seed-shaped supporting part 41b having the barbs 41c, and a tip of the gripping portion 42b constitutes the seed-shaped supporting part 41a.

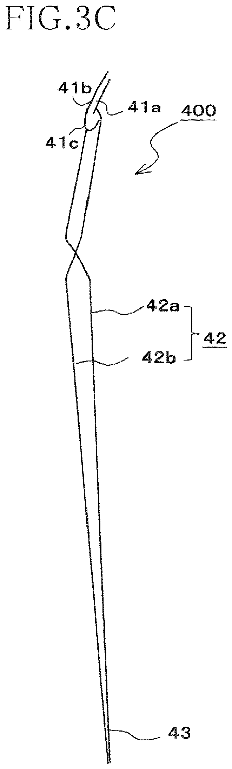

As shown in the side view of the placement assistant tool 400 of FIG. 3C, the other ends of the gripping portions 42a and 42b are joined together to constitute the supporting point 43 of the structure of tweezers. The supporting parts 41a and 41b are moved outwardly in a lateral direction by applying a force to the gripping portions 42a and 42b inwardly in a lateral direction so that the placement assistant tool 400 is brought to a state where the placement assistant tool 400 can pinch the ocular insert device 100. The ocular insert device 100 can be pinched by the supporting parts 41a and 41b by removing the force.

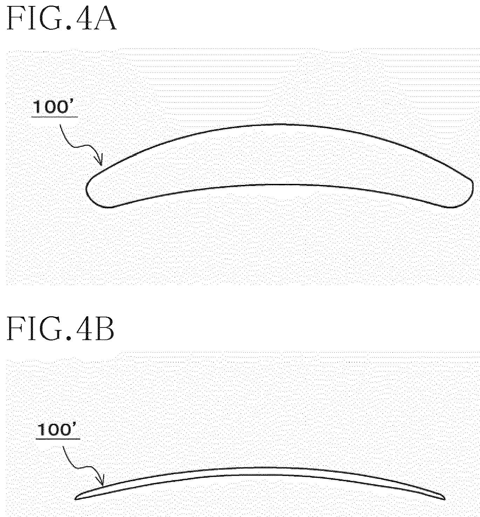

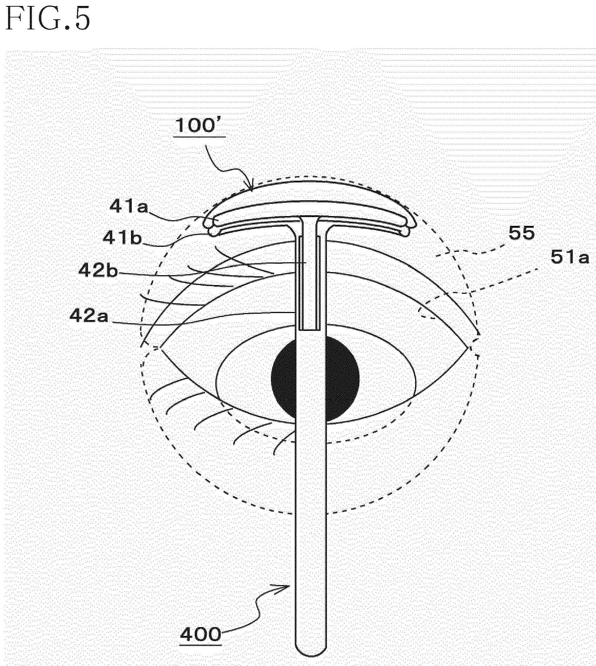

For example, there is a case where, as shown in a front view of FIG. 5, an ocular insert device 100' having an elongated seed shape as shown in a plan view of FIG. 4A and having a sheet shape as shown in a front view of FIG. 4B is located under the eyelid. In this case, a doctor pinches the ocular insert device 100' with the supporting parts 41a and 41b of the placement assistant tool 400 shown in FIG. 5 to place the ocular insert device 100' at the deep of the upper cul-de-sac 51a under the upper eyelid 55 of a patient. Then, the doctor applies a force to the gripping portions 42a and 42b to remove the ocular insert device 100' from the placement assistant device 400 and then remove the placement assistant device 400 from the upper cul-de-sac 51a. In this way, the ocular insert device 100' can be placed into the upper cul-de-sac 51a.

As described above, each of the ocular insert devices 100 and 100' as a first embodiment shown in FIGS. 1A to 5 includes the storage main body 1 that has a seed shape so as to be located under the eyelid and stores the drug, and the storage main body 1 has the held portion 1a in which a surface coming into contact with the eyelid is held by the tarsal plate 52.

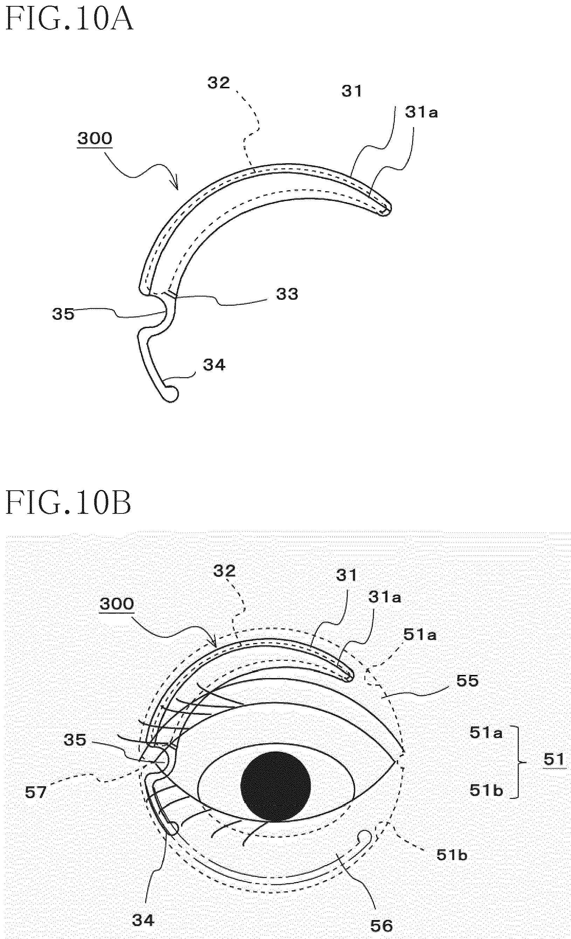

This configuration allows the storage main body 1 itself to be stably placed under the eyelid without depending on the function of a recess configured to avoid a frenulum 57, such as one shown in FIG. 10B. Since such a member configured to avoid the frenulum 57 is not necessary, the number of parts of the ocular insert device itself can also be reduced. Further, a larger volume of drug can be stored as compared to a conventional system without giving a feeling of insertion of a foreign body to a user. The amount of the drug can be increased as compared to a punctal plug type that is already commercially available.

It is to be noted that because the storage main body 1 is not exposed to the outside, the drug can be prevented from being deteriorated by light. Further, the concentration of the drug is not changed due to drying. Further, since the hollow potion 2 is provided by forming a cavity in the storage main body 1, an existing liquid drug can be directly used. This makes it possible to provide a highly-reliable general-purpose ophthalmic drug delivery system (DDS).

### <Second Embodiment>

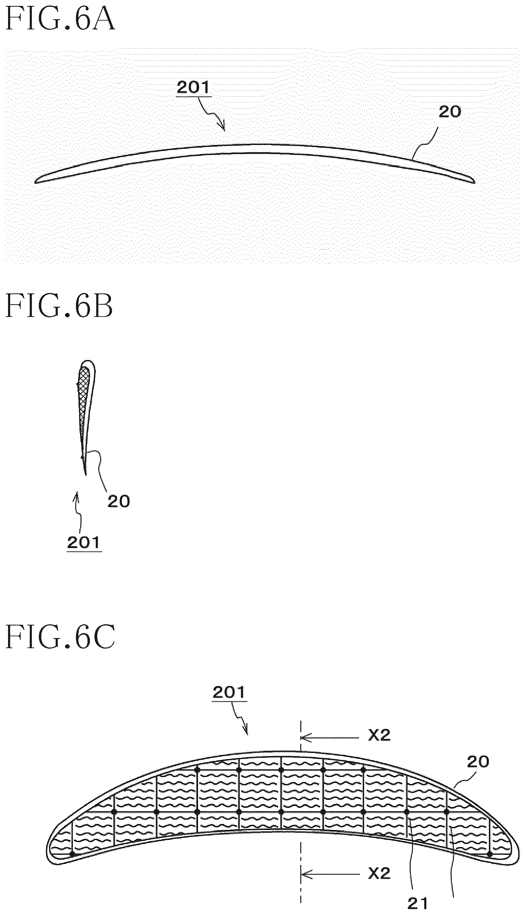

The following will describe ocular insert devices 201 to 203 as a second embodiment with reference to FIGS. 6A to 9. In this embodiment, a configuration for holding the storage main body 1 is not limited to the bulging shape, and a method is employed in which the storage main body 1 is stabilized using an anti-slipping function.

It is to be noted that, in consideration of a feeling of insertion of a foreign body in the anti-slipping function of a side of a surface facing the cornea 53 in a storage main body 20 (see FIG. 2), the anti-slipping function of the surface facing the cornea 53 may be much weaker than that of the surface facing the eyelid-side in the storage main body 20. Further, the thickness of the storage main body 20 may be reduced. For example, as shown in a front view of FIG. 6A, the ocular insert device 201 includes a sheet-shaped storage main body 20, and the surface of the storage main body 20 that comes into contact with the eyelid of a user is subjected to anti-slipping processing.

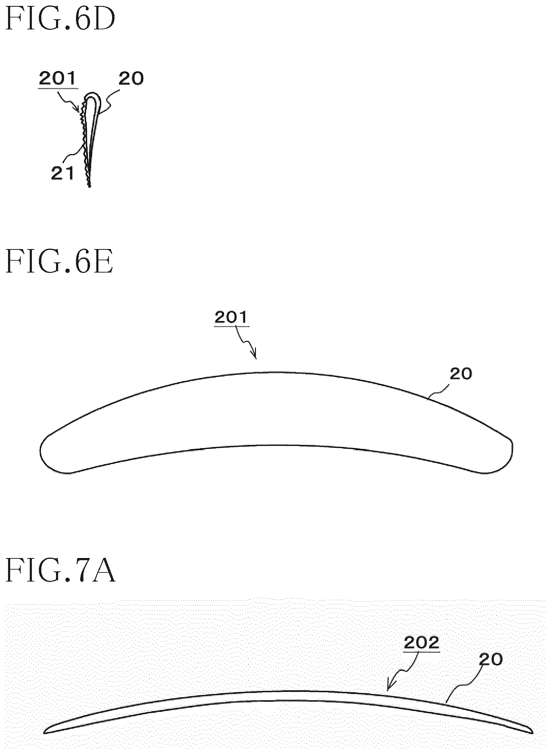

As shown in a side view of FIG. 6B, the ocular insert device 201 has a wing shape when viewed from its side. That is, as shown in a sectional view of FIG. 6D taken along the line X2-X2, the ocular insert device 201 has the wing-shaped cross section, but the cross-sectional shape of the ocular insert device 201 is not limited to a wing shape, and may be a circular, elliptical, oval, or comma shape as long as the ocular insert device 201 has a structure less likely to slip down. As shown in FIGS. 6C and 6D, the anti-slipping processing is performed so that one of the surfaces of the storage main body 20 has surface irregularities (rough surface) formed by, for example, a plurality of oblique lines, wavy lines, or straight lines, a geometric pattern, a pearskin pattern (Nashi-ji), or a combination of two or more of them. In this example, the storage main body 20 has a rough-surfaced portion 21 having a lattice wave pattern in which five wavy lines are formed in each of squares of a lattice. In the rough-surfaced portion 21, dots are provided at grid points. Such grid points may be omitted as long as the rough-surfaced portion 21 has a pattern less likely to slip down. It is to be noted that the other surface of the storage main body 20 shown in FIG. 6E is a surface that comes into contact with the cornea 53 (see FIG. 2), and is therefore subjected to mirror finishing.

In the ocular insert device 201, the friction coefficient between the surface subjected to the anti-slipping processing and the surface subjected to the mirror finishing can be increased, which makes the storage main body 20 less likely to slip down as compared to a case where any anti-slipping processing is not performed. This enables the storage main body 20to be stably placed between the outer periphery of the cornea 53 and the base of back side of the eyelid (in the conjunctival sac).

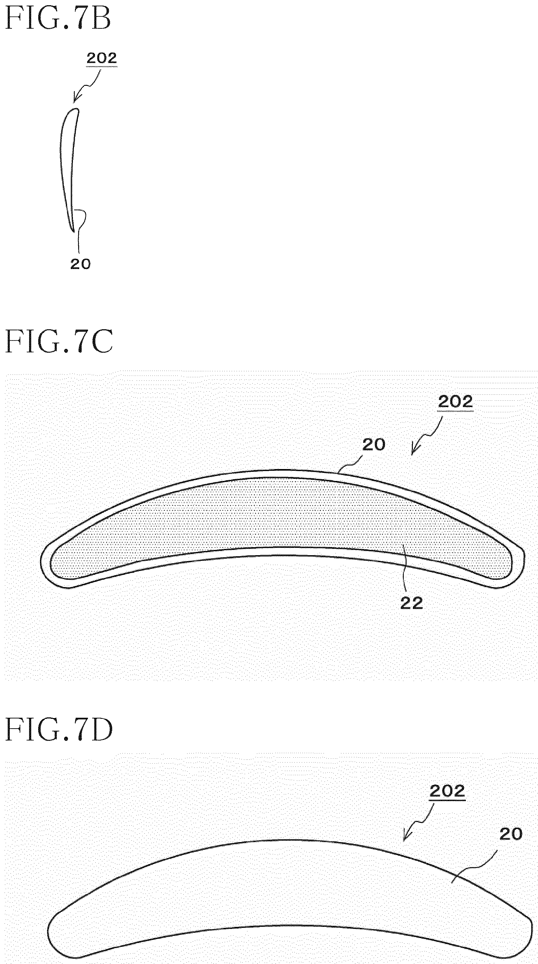

An ocular insert device 202 shown in FIG. 7A includes a storage main body 20. As shown in a side view of FIG. 7B, the storage main body 20 also has a wing-shaped side view. As shown in a plan view of FIG. 7C, a rough-surfaced portion 22 on the surface of the storage main body 20 has a pearskin pattern (Nashi-ji) or a pattern of small dots like fish roe (Nanako-ji). The rough-surfaced portion 22 is provided to increase a friction coefficient. A back surface of the storage main body 20 shown in FIG. 7D is subjected to the mirror finishing as in the case of the ocular insert device 201.

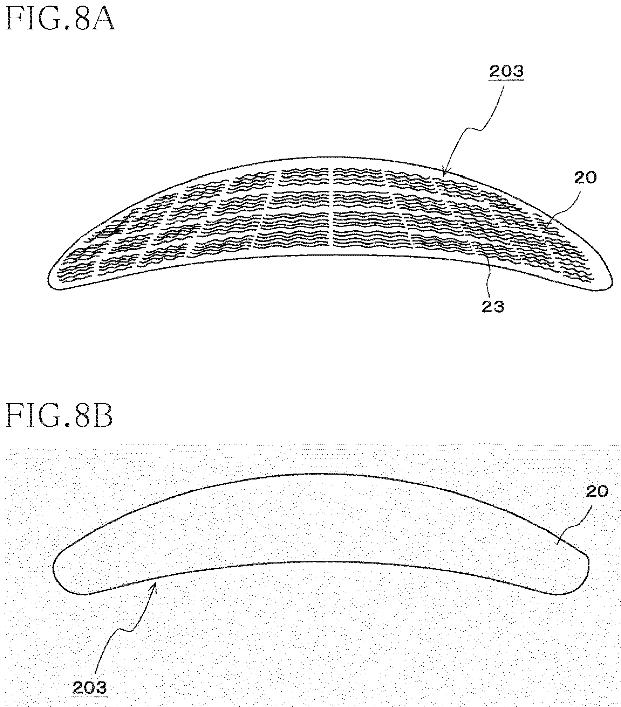

An ocular insert device 203 shown in FIG. 8A includes a storage main body 20. A pattern of grouped wavy lines is formed in a diffusion manner on the rough-surfaced portion 23 of the surface of the storage main body 20. The wavy lines are made longer on the lower side to achieve a high friction coefficient with the eyelid. As shown in FIG. 8B, a back surface of the storage main body 20 is subjected to the mirror finishing as in the case of the ocular insert devices 201 and 202 shown in FIGS. 6A to 7D.

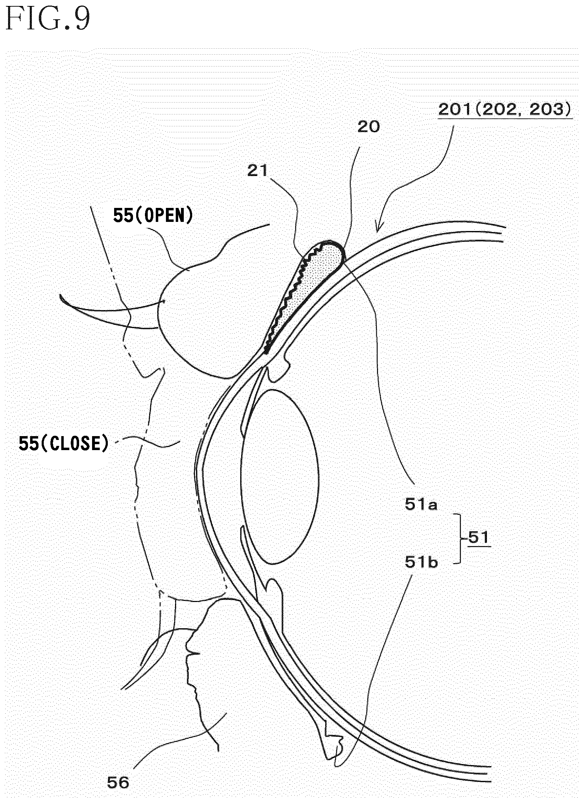

Here, examples of placing the ocular insert devices 201 to 203 will be described with reference to FIG. 9. For example, the ocular insert device 201 may be inserted into either the upper cul-de-sac 51a inside the upper eyelid 55 or the lower cul-de-sac 51b inside the lower eyelid 56, and when a high-concentration drug is required for treatment, the ocular insert devices 201 may be inserted into both the cul-de-sacs to control the concentration of the drug. As described above, for placing the ocular insert device 201, the placement assistant tool 400 can be used.

Since the upper eyelid 55 holds the rough-surfaced portion 21 of the storage main body 20 at the deep of the upper cul-de-sac 51a, when the eye is opened and closed, at a side of the upper eyelid 55, the upper eyelid 55 acts to prevent the storage main body 20 from slipping down. Further, since the eyeball-side surface of the storage main body 20 is subjected to the mirror finishing, it does not damages the eyeball. This allows the ocular insert device 201 to be placed into the conjunctival sac 51. It is to be noted that the ocular insert device 201 may be placed into the lower cul-de-sac 51b.

As described above, each of the ocular insert devices 201 to 203 as the second embodiment includes the storage main body 20 that has a seed shape so as to be located under the eyelid and that stores a drug, and the surface of the storage main body 20 that comes into contact with the eyelid of a user is subjected to the anti-slipping processing.

Such a configuration enables structure the storage main body 1 itself to be stably placed under the eyelid without depending on the function of a recess configured to avoid the frenulum 57, such as one shown in FIG. 10B. Further, it is possible to store a larger volume of drug as compared to a conventional system without giving a feeling of insertion of a foreign body to a user. Therefore, as in the case of the first embodiment, the amount of the drug can be increased as compared to a punctal plug type, and therefore the use time of the ocular insert device can be extended. Further, a member configured to avoid the frenulum 57 is not necessary, which also makes it possible to reduce the number of parts of the ocular insert device itself. Therefore, it is possible to provide a highly-reliable general-purpose ophthalmic drug delivery system (DDS) as in the case of the first embodiment.

It is to be noted that the ocular insert device 100' described with reference to FIGS. 4A to 5 includes not only the ocular insert device 100 according to the first embodiment but also the ocular insert device 201 to 203 described with reference to the second embodiment.

### <Third Embodiment>

The following will describe a configuration example of an ocular insert device 300 as a third embodiment with reference to FIGS. 10A and 10B. The ocular insert device 300 includes a storage main body 31. The storage main body 31 has a held portion 31a provided along its outer periphery and a hollow portion 32. The held portion 31a is formed to have a bulging shape so as to be held by the tarsal plate (not shown) when being placed into the upper cul-de-sac 51a. The hollow portion 32 has an opening 33 so that a drug can be exuded. From one end of the storage main body 31, an arc-shaped portion 34 is extended so as to be placed into the lower cul-de-sac 51b on a side of the lower eyelid 56. The arc shape of the arc-shaped portion 34 also plays a role in preventing the storage main body 31 from rotating in the conjunctival sac 51, and therefore the arc-shaped portion 34 works harmoniously with the held portion 31a, thereby enabling its posture to be maintained in a more stable position.

After the ocular insert device 300 is located under the eyelid as shown in FIG. 10B, the storage main body 31 is supported from a side of the lower cul-de-sac 51b toward a side of the upper cul-de-sac 51a dependently on a certain degree of tension. Further, a recess 35 is provided in a position that corresponds to the supporting point of the tension and is a starting point of the arc-shaped portion 34 to avoid the frenulum 57. It is to be noted that one end of the arc-shaped portion 34 is rounded to prevent damage to the eye.

Further, as shown by the chain double-dashed line in FIG. 10B, the posture of the storage main body 31 placed under the eyelid can be made most stable by extending the end of the above-mentioned arc-shaped portion 34 to a position facing the end of the storage main body 31. Preferably, the storage main body 31, the hollow portion 32, the arc-shaped portion 34, and the recess 35 may be integrally formed. This makes it possible to omit the held portion 1a (see FIG. 1B) described with reference to the first embodiment or the anti-slipping function or the like described with reference to the second embodiment.

As described above, in the ocular insert device 300 as the third embodiment, the arc-shaped portion 34 having a substantially semi-arc shape extends from one end of the storage main body 31 so that after the ocular insert device 300 is placed under the eyelid, it is configured that the storage main body 31 is supported from the side of the lower cul-de-sac 51b toward the side of the upper cul-de-sac 51a dependently on a certain degree of tension.

Such a structure enables the storage main body 31 to be stably placed in the conjunctival sac 51 in a deep position of the upper cul-de-sac 51a under the upper eyelid 55 without depending on the anti-slipping function unlike the first embodiment. The ocular insert device 300 can, of course, be used by turning it upside down.

Further, as in the case of the first and second embodiments, the hollow portion 32 is not exposed, to prevent a drug from being deteriorated by light. Further, the ocular insert device 300 is deeply located into the conjunctival sac 51, which also makes it possible to prevent the hollow portion 32 from being directly dried to deliver a drug at a stable concentration. This allows a large-capacity ophthalmic DDS to be provided as in the cases of the first and second embodiments.

It is to be noted that many modified examples according to the present invention have been described in detail with reference to the above embodiments, but other modified examples included in the technical scope of the present invention are easily conceivable by those skilled in the art based on the technical measures according to the present invention. The drawings illustrate specific embodiments, by which the present invention can be carried out, as examples. In this description, these embodiments are presented also as "examples". The drug and impregnated member described with reference to the storage bodies 1, 20, and 31 are examples thereof, and include new drugs and members that will be developed in the future.

### [INDUSTRIAL APPLICABILITY]

The present invention is very preferable to be applied to an ophthalmic DDS device that automatically supplies ophthalmic solutions to a site including the eyeball, the conjunctiva, etc.

### [EXPLANATION OF CODES]

- 1, 20, 31:: Storage main body
- 1a, 31 a:: Held portion
- 2, 32:: Hollow portion
- 3, 33:: Opening
- 21, 22, 23:: Rough-surfaced portion
- 34:: Arc-shaped portion
- 100, 201 to 203, 300:: Ocular insert device

## Claims

1. An ocular insert device comprising a storage main body made of a semi-arc-shaped piece, the storage main body having a predetermined width so as to be located under an eyelid and having a shape which extends along a portion where a palpebral conjunctiva and a bulbar conjunctiva are connected together so as to supply a drug, wherein the storage main body includes:
a held portion surface in which a surface thereof coming into contact with the eyelid is held, and
a surface thereof coming into contact with the eyeball with a smaller friction coefficient than that of the surface coming into contact with the eyelid.

2. The ocular insert device according to Claim 1, wherein the held portion is extended along an outer edge of the storage main body so as to form a bulging shape and is held by a tarsal plate.

3. The ocular insert device according to Claim 1, wherein the surface of the held portion that comes into contact with an eyelid includes a plurality of irregularities.

4. The ocular insert device according to any one of Claims 1 to 3, wherein the storage main body has a hollow portion, and
the hollow portion has a bag shape with a predetermined capacity and has at least one opening formed in a predetermined position to exude a drug.

5. The ocular insert device according to any one of Claims 1 to 3, wherein the storage main body is formed as an impregnated structure in which the storage main body itself is impregnated with a drug.
